# EUROPEAN PATENT APPLICATION

(11) **EP 4 129 160 A1**
(43) Date of publication of application: **08.02.2023**
(21) Application number: 21793824.0
(22) Date of filing: 01.04.2021
(51) Int. Cl.: A61B 5/00

(54) **BLOOD PRESSURE MEASUREMENT METHOD, APPARATUS, AND WEARABLE DEVICE**

(30) Priority: 20.04.2020 CN 202010313259
(71) Applicant: Huawei Technologies Co., Ltd., Shenzhen, Guangdong 518129 (CN)
(72) Inventor: KUANG, Yunsheng, Shenzhen, Guangdong 518129 (CN)
(74) Representative: Gill Jennings & Every LLP
(86) International application number: PCT/CN2021/084912
(87) International publication number: WO 2021/213170

(57) **Abstract**

A blood pressure measurement method and apparatus, and a wearable device are provided. The blood pressure measurement method includes: detecting a change of a user from a first state to a second state; starting measurement and inflating an airbag (160) to a preset pressure value at a preset rate if determining that the user has changed from the first state to the second state; obtaining first duration of an inflation process; determining a correction value based on the first duration; and after the measurement ends, obtaining a measurement value of a blood pressure, and correcting the measurement value based on the correction value, to obtain a final blood pressure value of the user. The blood pressure measurement method provides an early-morning blood pressure measurement manner in which the early-morning blood pressure can be automatically and accurately measured when the user is insensitive, thereby simplifying user's operations, and improving user experience.

## Description

This application claims priority to Chinese Patent Application No. 202010313259.7, filed with the China National Intellectual Property Administration on April 20, 2020, and entitled "BLOOD PRESSURE MEASUREMENT METHOD AND APPARATUS, AND WEARABLE DEVICE", which is incorporated herein by reference in its entirety.

### TECHNICAL FIELD

Embodiments of this application relate to the field of intelligent terminal technologies, and in particular, to a blood pressure measurement method and apparatus, and a wearable device.

### BACKGROUND

A blood pressure of a person fluctuates throughout the day, and a systolic blood pressure (SBP, Systolic Blood Pressure) (namely, a high pressure) and a diastolic blood pressure (DBP, Diastolic Blood Pressure) (namely, a low pressure) of a normal person show an obvious circadian rhythm. When a human body becomes awake from a deep sleep and starts to move, a blood pressure rapidly rises to a higher value from a lower one, and such a phenomenon is referred to as "morning surge" (mourning surge).

A rise of an early-morning blood pressure is a main factor for high incidences of cardiovascular and cerebrovascular events. Lowering the blood pressure in the morning can effectively reduce a probability of the cardiovascular and cerebrovascular events. Therefore, measurement of the early-morning blood pressure is crucial, through which the early-morning blood pressure may be monitored and an effective measure may be taken. In practical application, a blood pressure of a user measured within one hour after the user wakes up is considered as an early-morning blood pressure, and a closer measurement time to a waking time brings a more accurate result. Currently, a blood pressure of the user may be measured by using a wearable device, for example, a blood pressure wristband. However, because an existing blood pressure wristband does not have a function of tightness automatic adjustment, if the user tightly or loosely wears the wristband, a great deviation occurs in measurement of the early-morning blood pressure of the user. If the deviation is not corrected, the user may obtain incorrect measurement data, which adversely affects physical and mental health of the user.

### SUMMARY

Embodiments of this application provide a blood pressure measurement method and apparatus, and a wearable device, to provide a blood pressure measurement manner, improve blood pressure measurement accuracy, simplify user operations, and improve wearing experience of a user.

According to a first aspect, an embodiment of this application provides a blood pressure measurement method, including:

A change of a user from a first state to a second state is detected. Specifically, the first state may be an asleep state, and the second state may be an awake state. Because it is an early-morning blood pressure that is measured in this embodiment of this application, and the early-morning blood pressure is a blood pressure of the user within one hour after the user wakes up, it may be first detected whether the user is awake.

An airbag is inflated to a preset pressure value at a preset rate if it is determined that the user is awake. Specifically, if it is detected that the user is in an awake state, the airbag may be inflated to the preset pressure value at the preset inflation rate, to obtain a correction value.

The correction value is determined during inflation. Specifically, during inflation, a start moment of inflation may be first recorded; when a pressure value reaches a preset pressure threshold, an end moment is recorded; pressurization duration is obtained based on a difference between the end moment and the start moment; a current correction value may be obtained based on a mapping relationship between a preset pressurization duration and the correction value; and pressurization is continued to obtain a measurement value of a blood pressure.

After the measurement ends, the measurement value of the blood pressure is obtained, and the measurement value is corrected based on the correction value to obtain a final blood pressure value of the user. Specifically, a current measurement value of the user is obtained by measuring the early-morning blood pressure of the user. Because the current measurement value of the user may have a deviation due to tightness of a wearable device of the user, the measurement value needs to be corrected by using the correction value, where a corrected blood pressure value is a final early-morning blood pressure value.

In this implementation, after it is detected that the user is awake, measurement of the blood pressure of the user may be started, and the airbag is inflated at the preset rate. During inflation, the correction value may be determined. After the measurement ends, a measurement value of the blood pressure is obtained and is corrected by using the correction value, to obtain the final blood pressure value of the user. This provides a blood pressure measurement manner in which the early-morning blood pressure can be effectively and accurately measured when the user is insensitive, thereby improving accuracy of early-morning blood pressure measurement, simplifying user's operations, and improving wearing experience of the user.

In a possible implementation, the starting measurement and inflating an airbag to a preset pressure value at a preset rate if it is determined that the user has changed from the first state to the second state further includes:
obtaining a current pulse wave signal amplitude of the user if it is determined that the user has changed from the first state to the second state; and comparing the current pulse wave signal amplitude of the user with a target pulse wave signal amplitude, and if the current pulse wave signal amplitude of the user is not less than the target pulse wave signal amplitude, starting measurement and inflating the airbag to the preset pressure value at the preset rate. Specifically, after it is detected that the user is awake, a pulse wave signal amplitude of the user may be further detected. Through determining of the pulse wave signal amplitude, it may be determined whether an arm of the user is in a normal state, for example, whether the arm is compressed by the body. If the current pulse wave signal amplitude of the user matches the target pulse wave signal amplitude, blood pressure measurement may be started and the airbag is inflated to the preset pressure value at the preset rate.

In a possible implementation, the starting measurement and inflating an airbag to a preset pressure value at a preset rate if it is determined that the user has changed from the first state to the second state further includes:
if it is determined that the user has changed from the first state to the second state, detecting whether an acceleration in a direction opposite to gravity exists within a preset time; and if no acceleration in the direction opposite to gravity exists within the preset time, starting measurement and inflating the airbag to the preset pressure value at the preset rate. Specifically, after it is detected that the user is awake, it may be further detected whether an acceleration in the direction opposite to gravity exists. Through the detection of the acceleration in the direction opposite to gravity within the preset time, it may be determined whether a wrist and the heart of the user are at a same height level, where the wrist and the heart being at the same height level is a requirement for blood pressure measurement. The preset time may be a time point, for example, a moment at which it is detected that the user has changed from the first state to the second state. The preset time may alternatively be a time period, for example, a time period during which it is detected that the user changes from the first state to the second state. Therefore, if no acceleration in the direction opposite to gravity exists within the preset time, it indicates that the wrist and the heart of the user are at the same height level. In this case, blood pressure measurement may be started, and the airbag is inflated to the preset pressure value at the preset rate.

In a possible implementation, the obtaining first duration of the inflation process further includes:
recording a first start moment of inflating the airbag, and detecting a pressure value of the airbag during inflation; recording a first end moment when the pressure value reaches the preset pressure value; and determining the first duration based on a difference between the first end moment and the first start moment. Specifically, in the process of inflating the airbag, the start moment of inflation, namely, the first start moment, may be further recorded, and the pressure value in the airbag is detected. When the pressure value reaches the preset pressure threshold, the end moment, namely, the first end moment, is recorded. The pressurization duration, namely, the first duration, may be obtained based on the difference between the first end moment and the first start moment.

In a possible implementation, the method further includes:
comparing the first duration with each of a first duration threshold and a second duration threshold; sending a first signal if the first duration is less than or equal to the first duration threshold; and sending a second signal if the first duration is greater than the second duration threshold. Specifically, two duration thresholds, namely, the first duration threshold and the second duration threshold, may be preset. The two duration thresholds may be historical empirical values, where the first duration threshold may be a lower limit of duration, and the second duration threshold may be an upper limit of duration. If the pressurization duration obtained during inflation is less than or equal to the preset first duration threshold, it may be considered that the user tightly wears the wearable device. In this case, measurement may be suspended, and a tightly-worn signal, namely, the first signal, is sent to the user as a prompt. The signal may be sent in a voice broadcast manner or vibration manner. If the pressurization duration obtained during inflation is greater than the preset second duration threshold, it may be considered that the user loosely wears the wearable device. In this case, measurement may be suspended, and a loosely-worn signal, namely, the second signal, is sent to the user as a prompt. The signal may be sent in a voice broadcast manner or vibration manner. If the pressurization duration falls within a range between the preset first duration threshold and the preset second duration threshold, it is considered that the tightness of the wearable device of the user is appropriate, and the current inflation process is normal. In this case, pressurization is continued to complete blood pressure measurement.

In a possible implementation, the determining a correction value based on the first duration further includes:
determining a first slope based on the preset pressure value and the first duration; and determining the correction value based on the first slope. Specifically, the correction value may alternatively be obtained by using a pressurization slope (the first slope), where the pressurization slope may be obtained by using a quotient of the preset pressure threshold and the pressurization duration. After the pressurization slope is obtained, the correction value may be obtained based on a mapping relationship between the preset pressurization slope and the correction value.

In a possible implementation, the method further includes:
comparing the first slope with each of a first slope threshold and a second slope threshold; if the first slope is greater than or equal to the first slope threshold, sending a first signal; and if the first slope is less than the second slope threshold, sending a second signal. Specifically, two slope thresholds, namely, the first slope threshold and the second slope threshold may be preset, where the first slope threshold may be an upper limit of slope, and the second slope threshold may be a lower limit of slope. If the pressurization slope (the first slope) obtained during inflation is greater than or equal to the preset first slope threshold, it may be considered that the user tightly wears the wearable device. In this case, measurement may be suspended, and a tightly-worn signal, namely, the first signal, is sent to the user as a prompt. The signal may be sent in a voice broadcast manner or vibration manner. If the pressurization slope obtained during inflation is less than the preset second slope threshold, it may be considered that the user loosely wears the wearable device. In this case, measurement may be suspended, and a loosely-worn signal, namely, the second signal, is sent to the user as a prompt. The signal may be sent in a voice broadcast manner or vibration manner. If the pressurization slope falls within a range between the preset first slope threshold and the preset second slope threshold, it is considered that the tightness of the wearable device of the user is appropriate, and the current pressurization process is normal. In this case, the pressurization is continued to complete blood pressure measurement.

According to a second aspect, an embodiment of this application provides a blood pressure measurement apparatus, including:
a detection module, configured to detect a change of a user from a first state to a second state;
an inflation module, configured to start measurement and inflate an airbag to a preset pressure value at a preset rate if it is determined that the user has changed from the first state to the second state;
an obtaining module, configured to obtain first duration of an inflation process;
a correction module, configured to determine a correction value based on the first duration; and
an output module, configured to: after the measurement ends, obtain a measurement value of a blood pressure, and correct the measurement value based on the correction value, to obtain a final blood pressure value of the user.

In a possible implementation, the inflation module may include:
an obtaining unit, configured to obtain a current pulse wave signal amplitude of the user if it is determined that the user has changed from the first state to the second state;
an inflation unit, configured to compare the current pulse wave signal amplitude of the user with a target pulse wave signal amplitude, and if the current pulse wave signal amplitude of the user is not less than the target pulse wave signal amplitude, start measurement and inflate the airbag to the preset pressure value at the preset rate.

In a possible implementation, the inflation module may further include:
a detection unit, configured to, if it is determined that the user has changed from the first state to the second state, detect whether an acceleration in a direction opposite to gravity exists within a preset time; and
an inflation unit configured to, if no acceleration in the direction opposite to gravity exists within the preset time, start measurement and inflate the airbag to the preset pressure value at the preset rate.

In a possible implementation, the obtaining module may further include:
a recording unit, configured to: record a first start moment of inflation to the airbag, and detect a pressure value of the airbag during inflation; and record a first end moment when the pressure value reaches the preset pressure value; and
a calculating unit, configured to determine the first duration based on a difference between the first end moment and the first start moment.

In a possible implementation, the apparatus may further include:
a comparing module, configured to compare the first duration with each of a first duration threshold and a second duration threshold; and
a prompt module, configured to: send a first signal if the first duration is less than or equal to the first duration threshold; and send a second signal if the first duration is greater than the second duration threshold.

In a possible implementation, the correction module may include:
a calculating unit, configured to determine a first slope based on the preset pressure value and the first duration; and
a correction unit, configured to determine the correction value based on the first slope.

In a possible implementation, the apparatus may further include:
a comparing module, configured to compare the first slope with each of a first slope threshold and a second slope threshold; and
a prompt module, configured to: send a first signal if the first slope is greater than or equal to the first slope threshold; and send a second signal if the first slope is less than the second slope threshold.

According to a third aspect, this application provides a wearable device, including a processor, a signal receiver, an air pump control circuit, an air pump, an airbag, and a pressure sensor, where
the signal receiver is configured to detect a PPG signal and send the PPG signal to the processor;
the pressure sensor is configured to detect a pressure value of the airbag in a process of inflating the airbag by the air pump, and send the pressure value to the processor; and
the processor is configured to: determine, based on the PPG signal, that the user has changed from a first state to a second state, indicate the air pump control circuit to drive the air pump to inflate the airbag to the preset pressure value at a preset rate, and obtain first duration of the inflation process; determine a correction value based on the first duration; and after the measurement ends, obtain a measurement value of a blood pressure, and correct the measurement value based on the correction value, to obtain a final blood pressure value of the user.

In a possible implementation, the signal receiver is further configured to obtain a current pulse wave signal amplitude of the user.

The processor is further configured to: compare the current pulse wave signal amplitude of the user with a target pulse wave signal amplitude; and if the current pulse wave signal amplitude of the user is not less than the target pulse wave signal amplitude, notify the air pump control circuit to drive the air pump to inflate the airbag to the preset pressure value at the preset rate.

In a possible implementation, the wearable device further includes a motion sensor, where
the motion sensor is configured to detect whether an acceleration in a direction opposite to gravity exists within a preset time, and send a detection result to the processor.

The processor is further configured to: determine that no acceleration in the direction opposite to gravity exists within the preset time, and notify the air pump control circuit to drive the air pump to inflate the airbag to the preset pressure value at the preset rate.

In a possible implementation, the processor is further configured to record a first start moment and a first end moment of inflating the airbag by the air pump, and determine the first duration based on a difference between the first end moment and the first start moment.

In a possible implementation, the wearable device further includes an audio circuit and a vibrator, where
the audio circuit is configured to give a voice prompt; and
the vibrator is configured to vibrate for prompt.

The processor is further configured to compare the first duration with each of a first duration threshold and a second duration threshold.

If the first duration is less than or equal to the first duration threshold, the processor indicates the audio circuit and/or the vibrator to send a first signal.

If the first duration is greater than the second duration threshold, the processor indicates the audio circuit and/or the vibrator to send a second signal.

In a possible implementation, the processor is further configured to determine a first slope based on the preset pressure value and the first duration, and determine the correction value based on the first slope.

In a possible implementation, the wearable device further includes an audio circuit and a vibrator, where
the audio circuit is configured to give a voice prompt; and
the vibrator is configured to vibrate for prompt.

The processor is further configured to compare the first slope with each of a first slope threshold and a second slope threshold.

If the first slope is greater than or equal to the first slope threshold, the processor indicates the audio circuit and/or the vibrator to send a first signal.

If the first slope is less than the second slope threshold, the processor indicates the audio circuit and/or the vibrator to send a second signal.

### BRIEF DESCRIPTION OF DRAWINGS

FIG. 1 is a schematic diagram of a structure of an electronic device according to an embodiment of this application;
FIG. 2 is a flowchart of a blood pressure measurement method according to an embodiment of this application;
FIG. 3 is a diagram of a mapping relationship between a blood pressure offset and a pressurization duration according to an embodiment of this application;
FIG. 4 is a diagram of a mapping relationship between a blood pressure offset and a pressurization slope according to an embodiment of this application; and
FIG. 5 is a schematic diagram of a structure of a blood pressure measurement apparatus according to an embodiment of this application.

### DESCRIPTION OF EMBODIMENTS

Terms used in embodiments of this application are only used to explain specific embodiments of this application, but are not intended to limit this application.

In the conventional technology, during measurement of an early-morning blood pressure of a user, a wearable device, for example, a blood pressure wristband or a blood pressure watch cannot automatically detect tightness of wearing, and cannot correct a measurement value of the blood pressure based on the tightness of current wearing, thereby causing the measurement value of the blood pressure deviates greatly from an actual value.

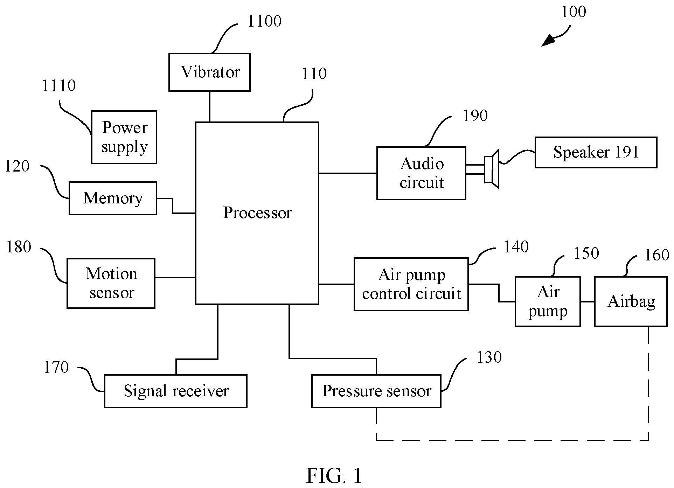

FIG. 1 is a schematic diagram of a structure of an electronic device 100 according to an embodiment of this application. The electronic device 100 may include a processor 110, a memory 120, a pressure sensor 130, an air pump control circuit 140, an air pump 150, an airbag 160, and a signal receiver 170. The processor 110, the memory 120, the pressure sensor 130, the air pump control circuit 140, and the signal receiver 170 may communicate with each other through an internal connection path, to transfer control and/or data signals. The memory 120 may be configured to store a computer program, and the processor 110 may be configured to invoke and run the computer program from the memory 120. In a specific implementation, the processor 110 may be a micro-controller unit (Micro-Controller Unit, MCU). The memory 120 may be a buffer (buffer), and the memory 120 may be further configured to store historical data, where the historical data may include a static pressure range corresponding to an effective pulse wave signal during blood pressure measurement and a corresponding static pressure value point when each pulse wave feature occurs. The pressure sensor 130 may be connected to the airbag 160 through a connection hole or a catheter, and is configured to obtain a pressure value of the airbag 160 in real time. The air pump control circuit 140 may be configured to output an inflation voltage to the air pump 150, to control a rate at which the air pump 150 inflates the airbag 160. A larger inflation voltage indicates a larger rate of inflation by the air pump 150 and a larger pressurization rate of the airbag 160. The air pump 150 may also be connected to the airbag 160 through a connection hole or a catheter, to inflate or deflate the airbag 160. The airbag 160 may be made of three materials: polyvinyl chloride (Polyvinyl Chloride, PVC), thermoplastic polyurethanes (Thermoplastic polyurethanes, TPU), and silicon. The airbag 160 is flat when in a normal condition without inflation. Once being inflated, the airbag 160 slowly bulges and compresses the radial artery of the wrist, so that the signal receiver 170 extracts a pulse wave signal. The signal receiver 170 is configured to detect a pulse wave signal of the user, where the pulse wave signal may be obtained through photoplethysmography (PPG, photoplethysmography).

The memory 120 may be a read-only memory (read-only memory, ROM) or another type of static storage device that can store static information and instructions, or a random access memory (random access memory, RAM) or another type of dynamic storage device that can store information and instructions, or may be an electrically erasable programmable read-only memory (electrically erasable programmable read-only memory, EEPROM), a compact disc read-only memory (compact disc read-only memory, CD-ROM) or another optical disk storage, an optical disc storage (including a compact disc, a laser disc, an optical disc, a digital versatile disc, a Blu-ray disc, or the like), a disk storage medium or another magnetic storage device, or any other medium that can be used to carry or store expected program code in a form of instructions or a data structure and that can be accessed by a computer.

Optionally, the electronic device 100 may further include a motion sensor 180. The motion sensor 180 may be configured to detect actions of limbs of the user. The motion sensor 180 may be an acceleration (ACC, accelerator) sensor, or may be a motion sensor of another type. This is not limited in this embodiment of this application.

The processor 110 and the memory 120 may be combined into one processing apparatus, or more commonly, are independent of each other. The processor 110 may be configured to execute program code stored in the memory 120 to implement the foregoing functions. In a specific implementation, the memory 120 may also be integrated into the processor 110, or may be independent of the processor 110.

In addition, to make the electronic device 100 more versatile in function, one or more of an audio circuit 190 and a vibrator 1100 may further be disposed in the electronic device 100. The audio circuit may further include a speaker 191. The audio circuit 190 and the speaker 191 may be configured to give a voice prompt, to prompt the user to adjust a posture, so that the device measures a blood pressure of the user. The vibrator 1100 may be configured to vibrate, to prompt the user to adjust a posture, so that the device measures a blood pressure of the user.

Optionally, the electronic device 100 may further include a power supply 1110, configured to provide power to various components or circuits in the electronic device 100.

It should be understood that the processor 110 in the electronic device 100 shown in FIG. 1 may be a system-on-chip SOC, and the processor 110 may include a central processing unit (Central Processing Unit, CPU for short below), and may further include a processor of another type, for example, a graphics processing unit (Graphics Processing Unit, GPU for short below).

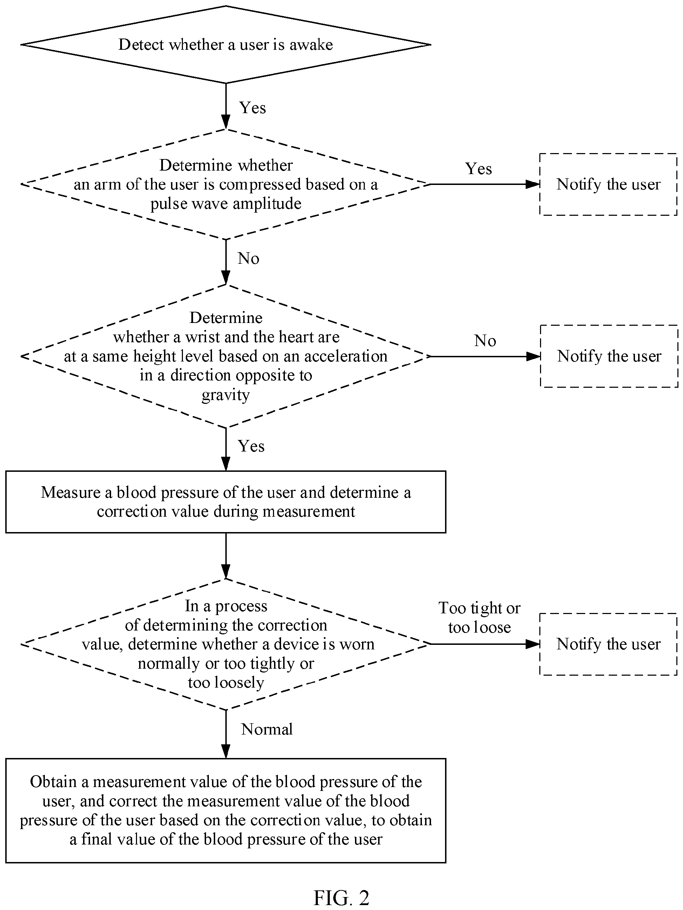

A blood pressure measurement method provided in this application is described herein with reference to FIG. 2 to FIG. 4. The method may be applied to the electronic device 100.

FIG. 2 is a flowchart of a blood pressure measurement method according to an embodiment of this application. As shown in FIG. 2, the blood pressure measurement method may include the following steps:

Step 101: Detect a current sleep status of a user.
Specifically, the sleep status of the user may be divided into two types in advance: an asleep state and an awake state. Because it is an early-morning blood pressure that is measured in this embodiment of this application, and the early-morning blood pressure is a blood pressure of the user within one hour after the user wakes up, the sleep status of the user may be detected first. The detection may be continuous detection in the early morning, where the early morning may be a specified time period from a preset time point. For example, if the time of 5:00 a.m. is preset as a start time point of early morning, the sleep status of the user is continuously detected from 5:00 a.m. until it is detected that the user wakes up. The continuous detection may be continuous detection on the sleep status of the user, for example, continuous detection may be performed in a time period. In addition to the continuous detection, the detection may be performed at intervals, for example, detection is performed on the user every 5 minutes.

A manner of detecting whether the user is awake may be performed by using a signal of the motion sensor 180, or may be performed by using a photoplethysmography (PPG, photoplethysmography) signal, where the PPG signal may be obtained by detection by the signal receiver 170. When the signal of the motion sensor 180 is used to detect whether the user is awake, because when the user is in the asleep state, the user's hand does not have continuous or high-intensity actions, but once the user wakes up, continuous actions occur, the motion sensor 180 may detect these actions to determine whether the user is awake. When the PPG signal of the signal receiver 170 is used to detect whether the user is awake, because a feature of a pulse wave varies accordingly with the status of the user from the asleep state to the awake state, whether the user is awake may be detected by detecting a corresponding change of the feature of the pulse wave by using the PPG signal. Further, to improve accuracy and timeliness of detecting whether the user is awake, the signal of the motion sensor 180 and the PPG signal of the signal receiver 170 may be combined to determine whether the user is awake.

Step 102: If it is detected that the current sleep status of the user is an awake state, measure a blood pressure of the user, and determine a correction value during measurement.

Specifically, if it is detected at any moment that the user is currently in an awake state, the processor 110 may send an inflation signal to the air pump control circuit 140, to start measurement of the blood pressure of the user. After receiving the inflation signal, the air pump control circuit 140 starts the air pump 150. The air pump 150 continuously inflates the airbag 160 and the pressure sensor 130 detects a pressure value in the airbag 160 in real time. A rate at which the air pump 150 inflates the airbag 160 may be preset, and the inflation rate may be an empirical value. When the pressure value reaches a target pressure value, the processor 110 may determine a correction value for the wearable device of the user by calculating inflation duration. The wearable device may include a blood pressure wristband, a blood pressure watch, or another type of wearable blood pressure measurement device. During a specific implementation, the target pressure value may be preset, and the pressure sensor 130 integrated in a wearable device body of the user may detect, in the process of inflating the airbag 160, the pressure value in the airbag 160 in real time, and determine whether the pressure value reaches the target pressure value. The target pressure value may be usually set based on a critical pressure value when a pulse wave signal appears. At initial inflation moments, a pressure exerted by the airbag on the radial artery is too small to have a compression effect on the radial artery. As a result, within a time period after inflation or when an initial pressure value is below a specific pressure value, no pulse wave signal can be extracted. In addition, no pulse wave signal occurs on anyone at a pressure below a specific pressure value. Generally, a critical pressure value of a pulse wave signal is 20 mmHg to 30 mmHg. Therefore, in this embodiment of this application, the target pressure value may be set to 20 mmHg, or another target pressure value, for example, 30 mmHg. This is not limited in this embodiment of this application.

The air pump 150 may be integrated into the wearable device body of the user, is in connection with a nozzle of the airbag 160 via a catheter, and inflates the airbag 160, so that the pressure in the airbag 160 rises, thereby achieving pressurization. After blood pressure measurement ends, air in the airbag 160 may also be released through the air pump 150. The airbag 160 functions like a cuff of an upper arm sphygmomanometer, is fixed on a wrist of a human body, presses the radial artery to be closed when being inflated and pressurized, and transmits a pressure signal through the pressure sensor 130 during the pressurization. A pulse wave signal and a static pressure signal are extracted based on the pressure signal to measure a blood pressure. The airbag 160 typically have two nozzles, one of which is connected to the air pump 150 and the other is connected to the pressure sensor 130.

Optionally, after it is detected that the user is awake, the speaker 191 may give a voice prompt, or the vibrator 1100 vibrates, to prompt the user to adjust a posture, so that the device measures the blood pressure of the user. In a possible manner, after the user is prompted through voice or vibration, a time period may be further preset. In other words, measurement of the blood pressure of the user may be started after the time period, so that the user has time to adjust the posture. The preset time period may be an empirical value, for example, 15s. This is not limited in this embodiment of this application.

Optionally, after it is detected that the user is awake, a current posture of the user may be further detected. During measurement of an early-morning blood pressure of the user, the user is usually lying with some postures that may not be suitable for blood pressure measurement, for example, an arm is compressed by the body. Therefore, an inappropriate posture may cause a deviation in early-morning blood pressure measurement. If an abnormal posture of the user is detected, measurement of the early-morning blood pressure of the user may be suspended.

During detection of the current posture of the user, determining may be performed by using a pulse wave signal amplitude, where the pulse wave signal amplitude may be used to detect arm compression. During a specific implementation, a basic requirement for wrist blood pressure measurement is that peripheral arteries (including brachial artery, radial artery, and ulnar artery) of a measured arm should not be compressed and keep a blood flow unobstructed. However, when the user is in a lying position, the arm tends to be squeezed by the body (for example, lateral lying), causing compression on the upper arm artery and obstruction of blood flow, and affecting blood pressure measurement. In this case, the pulse wave signal amplitude may be detected by using the PPG signal of the signal receiver 170 to determine whether the upper arm blood pressure is compressed. The signal receiver 170 may usually be integrated on a side of the wearable device close to the skin of the user to transmit and receive light intensity signals (a light source may be in a plurality of forms, such as red light, green light, and infrared light), to obtain a pulse wave signal of the wrist. Therefore, after the signal receiver 170 detects the pulse wave signal, the processor 110 may calculate an amplitude of the pulse wave signal, and then compare the current pulse wave signal amplitude of the user with a target pulse wave signal amplitude, or compare the current pulse wave signal amplitude of the user with a pulse wave signal amplitude threshold which is preset and pre-stored in the memory 120. The pulse wave vibration amplitude usually reflects an intensity of heart beating. Typically, in a case that peripheral arteries of the upper arm (including brachial artery, radial artery, ulnar artery, and the like) are not compressed, a pulse wave vibration amplitude of a human body does not change greatly, but once the arteries are compressed, blood flow is obstructed, and the pulse wave vibration amplitude decreases. Therefore, if it is found that the pulse wave signal amplitude is less than the target pulse wave signal amplitude, it indicates that the pulse wave signal amplitude decreases, and it may be determined that the measured arm is compressed. At this time, it is not suitable to perform blood pressure measurement. If the pulse wave signal amplitude is not less than the target pulse wave signal amplitude, it may be considered that a current posture of the user is normal, and blood pressure measurement may be performed.

Optionally, during detection of the current posture of the user, determining may alternatively be performed by detecting an acceleration in a direction opposite to gravity. In other words, it is detected whether an acceleration in a direction opposite to gravity exists, to determine whether the posture of the user is normal. During a specific implementation, it is required for blood pressure measurement that a wrist and heart need to be kept at the same height level. When the user lies flat, the wrist and heart are usually at the same height level. However, there are some postures, for example, stretching in which the wrist is at a level higher than the heart, and such postures are very likely to occur at wake-up moments. Therefore, the motion sensor 180 detects whether an acceleration in the direction opposite to gravity exists, to determine whether the wrist and the heart are at the same height level, and further determine whether it is suitable to measure the blood pressure of the user. Generally, an action with the wrist at a level higher than the heart is accompanied by a motion process. In this process, the motion sensor 180 detects acceleration components in directions of three axes in real time. For an action with the wrist at a level higher than the heart, there must be an acceleration component in the direction opposite to gravity. When it is reflected on the three axes of the motion sensor 180, an acceleration in the direction opposite to gravity appears after the acceleration components of gravity in the direction of the three axes are combined. Such feature is used to determine whether the wrist is at the same height level with the heart, to determine whether the posture of the user is normal, that is, determine whether it is suitable to measure the blood pressure of the user. In addition, to ensure normal execution of the device, a time may be preset in a process of detecting an acceleration in the direction opposite to gravity, to avoid long-time detection of the acceleration in the direction opposite to gravity. During a specific implementation, the preset time may be a time point. For example, the acceleration in the direction opposite to gravity is detected at a moment when it is detected that the user has changed from a first state to a second state. If no acceleration in the direction opposite to gravity is detected at the moment, blood pressure measurement may be continued. The preset time may alternatively be a time period. For example, the acceleration in the direction opposite to gravity is continuously detected within a time period after it is detected that the user has change from the first state to the second state. If no acceleration in the direction opposite to gravity is detected within the time period, detection of an acceleration in the direction opposite to gravity is no longer performed, and blood pressure measurement is performed.

It should be understood that, during a specific implementation of the foregoing two steps of detecting the pulse amplitude and detecting an acceleration in the direction opposite to gravity, one of the steps may be randomly selected, or the two steps may be performed. If the two steps are performed, the two steps are not limited to a specific execution sequence. This is not limited in this embodiment of this application.

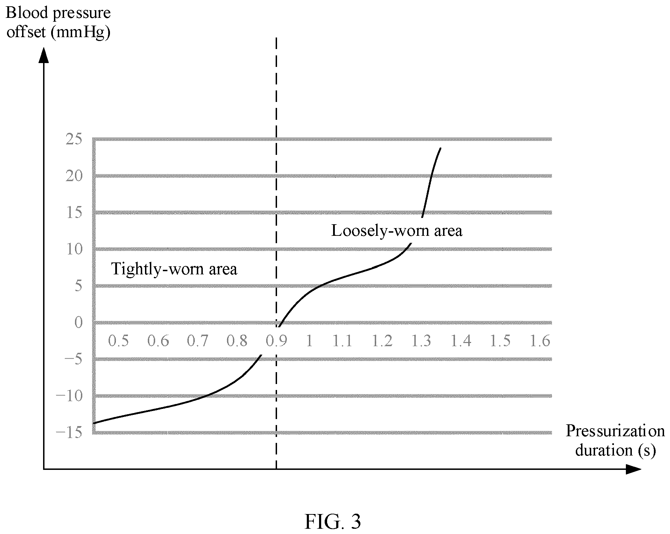

Further, after the pressure value of the airbag 160 reaches the target pressure value, a correction value may be further determined based on duration accumulated from a moment of initial inflation to a moment at which the target pressure value is reached. During a specific implementation, a start moment may be recorded at initial inflation. Then the pressurization may be ended when the pressure value reaches the target pressure value, and an end moment is recorded. The pressurization duration may be obtained based on the start moment and the end moment. It should be understood that, if the user loosely wears the wearable device, a space exists between the airbag and the skin. After pressurization starts, the airbag 160 needs to bulge for a period of time before contacting the skin. As a result, a pulse wave signal moves towards a high pressure, and a measured blood pressure value is greater than an actual blood pressure value. On the contrary, if the device is tightly worn, the pulse wave signal moves towards low pressure, and a measured blood pressure value is less than an actual blood pressure value. A blood pressure offset and the pressurization duration have a mapping relationship, where the blood pressure offset may be the correction value. As shown in FIG. 3, a thermoplastic polyurethanes (Thermoplastic polyurethanes, TPU) airbag with a width of 28 mm is used as an example. As pressurization duration increases, a blood pressure offset increases in proportion to the pressurization duration. However, an actual measured blood pressure value is higher than an actual blood pressure value, and the blood pressure offset increases as the pressurization duration increases and is a positive number, which also means that the user loosely wears the wearable device. Similarly, as the pressurization duration decreases, an actual measured blood pressure value is lower than an actual blood pressure value, and the blood pressure offset decreases continuously as the pressurization duration decreases and is a negative number. A point with an offset of 0 is an optimal wearing point, which means that measurement has no deviation, and any point that offsets the point will produce a deviation.

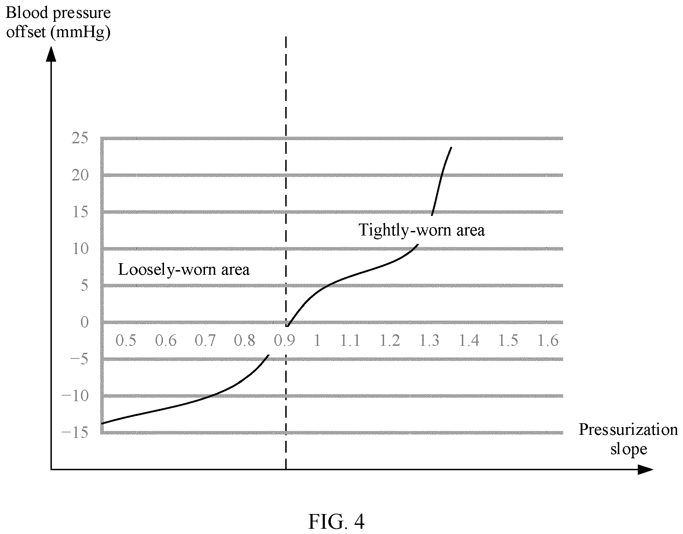

Optionally, the correction value may be obtained based on a pressurization slope, which may be obtained through calculation of the pressurization duration and a target pressure value, for example, a pressurization slope may be a quotient of the target pressure value and the pressurization duration. FIG. 4 is a diagram of a mapping relationship between a pressurization slope and a blood pressure offset. The blood pressure offset also increases as the pressurization slope increases and is a positive number, and decreases as the pressurization slope decreases and is a negative number. A positive value range of the blood pressure offset means that the user tightly wears the wearable device, and a negative value range of the blood pressure offset means that the user loosely wears the wearable device.

In a possible manner, during determining the correction value, current wearing tightness of the wearable device of the user may be further determined based on pressurization duration. For example, standard duration may be preset. If the pressurization duration is greater than the standard duration, it indicates that the pressurization duration is greater than actual duration, and the device is loosely worn. If the pressurization duration is less than or equal to the standard duration, it indicates that the pressurization duration is less than actual duration, and the device is tightly worn.

During a specific implementation, first standard duration and second standard duration may be preset first, where the first standard duration may be used to determine whether the device is tightly worn, and the second standard duration may be used to determine whether the device is loosely worn. A start moment may be recorded at initial pressurization. Then the pressurization may be ended when the pressure value reaches the target pressure value, and an end moment is recorded. The pressurization duration may be obtained based on the start moment and the end moment. The pressurization duration may be compared with each of the first standard duration and the second standard duration. If the pressurization duration is less than or equal to the first standard duration, it indicates that the device is tightly worn, that is, the wearing tightness is at a tight degree; and if the pressurization duration is greater than the second standard duration, it indicates that the device is loosely worn, that is, the wearing tightness is at a loose degree. If the pressurization duration is neither less than the first standard duration nor greater than the second standard duration, the correction value may be determined based on the pressurization duration.

Optionally, the wearing tightness may be further determined based on the pressurization slope. During a specific implementation, two standard slopes: a first standard slope and a second standard slope may be preset first, where the first standard slope may be used to determine whether the device is tightly worn, and the second standard slope may be used to determine whether the device is loosely worn. A start moment may be recorded at initial inflation. Then the pressurization may be ended when the pressure value reaches the target pressure value, and an end moment is recorded. The pressurization slope may be obtained based on the start moment and the end moment. The pressurization slope may be compared with each of the first standard slope and the second standard slope. If the pressurization slope is greater than or equal to the first standard slope, it indicates that the device is tightly worn, that is, the wearing tightness is at a tight degree; and if the pressurization slope is less than the second standard slope, it indicates that the device is loosely worn, that is, the wearing tightness is at a loose degree. If the pressurization duration is neither greater than nor equal to the first standard slope nor less than the second standard slope, the correction value may be determined based on the pressurization slope.

Further, after the wearing tightness is obtained, a prompt signal may be further sent, where the prompt signal may be used to indicate the wearing tightness to the user. For example, if the wearing tightness is at a tight degree, a tightly-worn signal may be sent; if the wearing tightness is at a loose degree, a loosely-worn signal may be sent, to prompt the user to make adjustment. The signal may be sent by the speaker 191 through a voice prompt, or may be sent by the vibrator 1100 through a prompt by vibration. This is not limited in this embodiment of this application.

Step 103: Obtain a measurement value of the blood pressure of the user, and correct the measurement value of the blood pressure of the user based on the correction value, to obtain a final blood pressure value of the user.

Specifically, a current measurement value of the user may be obtained by measuring an early-morning blood pressure of the user. Because the current measurement value of the user may have a deviation due to tightness of the wearable device of the user, the current measurement value needs to be corrected by using the correction value, where a corrected blood pressure value is the final early-morning blood pressure value.

The blood pressure is usually measured by extracting a pulse wave signal, where the extraction of the pulse wave signal is usually by filtering. Therefore, a Butterworth filter or a finite impulse response (FIR, Finite Impulse Response) filter may be used. Generally, an envelope of the extracted pulse wave signal shows a single peak which features an increase followed by a decrease in values, but pulse wave envelopes of a small quantity of people show double peaks or other waveforms. After the pulse wave signal is extracted, features of the pulse wave signal can be extracted, including a peak pressure, a maximum slope of static pressure, and the like. These features are all related to the blood pressure value, so that the blood pressure value can be calculated by extracting these features. For example, a diagram of a mapping relationship between a static pressure and a pulse wave can be obtained according to a static pressure curve and a pulse wave curve, a static pressure value corresponding to a highest peak value can be found in the diagram, and the blood pressure value can be obtained by using the static pressure value.

Using FIG. 3 as an example, in an area in which the wearable device of the user is loosely worn, the offset is a positive value. In this case, the offset value may be subtracted from the current measurement value to obtain a final measurement value. Similarly, in an area in which the wearable device of the user is tightly worn, the offset is a negative value. In this case, the offset value may be subtracted from the current measurement value to obtain a final measurement value. In this way, the current measurement value may be corrected based on the offset, to obtain a final measurement value without a deviation. It should be noted that deviation curves obtained are different due to different materials, widths, and structures of airbags. Therefore, a mapping relationship between the blood pressure offset and the pressurization duration may be determined based on a hardware specification of the wearable device of the user.

It should be understood that the blood pressure of the user is classified into systolic blood pressure and diastolic blood pressure, and the blood pressure may be usually measured by using an oscilloscope method. According to the oscilloscope method, any blood pressure measurement device corresponds to two coefficients, namely, a systolic blood pressure coefficient and a diastolic blood pressure coefficient. After pressure measurement is performed by the blood pressure measurement device, a diagram of a mapping relationship between a static pressure value and a pulse wave can be obtained. In the diagram, a static pressure value corresponding to a highest peak value can be found, the systolic blood pressure can be obtained by multiplying the static pressure value by the systolic blood pressure coefficient, and the diastolic blood pressure can be obtained by multiplying the static pressure value with the diastolic blood pressure coefficient. Therefore, there are two blood pressure offsets of the user, namely, an SBP offset and a DBP offset, and it also means that there is a mapping relationship between the SBP offset and the pressurization duration and a mapping relationship between the DBP offset and the pressurization duration. It can be learned that two measurement values, namely, an SBP measurement value and a DBP measurement value, are obtained from measurement on an early-morning blood pressure of the user. The SBP measurement value, after being corrected by using the SBP offset, is a final SBP measurement value. The DBP measurement value, after being corrected by using the DBP offset, is a final DBP measurement value. The final SBP measurement value and the final DBP measurement value are final measurement values of the early-morning blood pressure of the user.

In this embodiment, after it is detected that the user wakes up, the wearable device of the user is inflated to determine the correction value of the wearable device of the user. Then, an early-morning blood pressure of the user is measured and a measurement value of the blood pressure is corrected by using the correction value. This provides a manner of measuring the early-morning blood pressure in which the early-morning blood pressure can be effectively and accurately measured without being perceived by the user, improving accuracy of early-morning blood pressure measurement, simplifying user's operations, and improving wearing experience of the user.

It can be understood that some or all of the steps or operations in the foregoing embodiments are merely examples, and other operations or variants of various operations may be further performed in this embodiment of this application. In addition, the steps may be performed in another order different from that presented in the foregoing embodiments, and not all the operations in the foregoing embodiments may be performed.

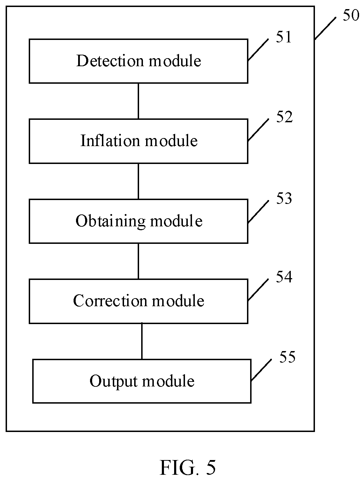

FIG. 5 is a schematic diagram of a structure of a blood pressure measurement apparatus according to an embodiment of this application. As shown in FIG. 5, the blood pressure measurement apparatus 50 may include: a detection module 51, an inflation module 52, an obtaining module 53, a correction module 54, and an output module 55.

The detection module 51 is configured to detect a change of a user from a first state to a second state.

The inflation module 52 is configured to start measurement and inflate an airbag to a preset pressure value at a preset rate if it is determined that the user has changed from the first state to the second state.

The obtaining module 53 is configured to obtain first duration of an inflation process.

The correction module 54 is configured to determine a correction value based on the first duration.

The output module 55 is configured to, after the measurement ends, obtain a measurement value of a blood pressure, and correct the measurement value based on the correction value, to obtain a final blood pressure value of the user.

In a possible implementation, the inflation module 52 may include an obtaining unit 521 and an inflation unit 522.

The obtaining unit 521 is configured to obtain a current pulse wave signal amplitude of the user if it is determined that the user has changed from the first state to the second state.

The inflation unit 522 is configured to: compare the current pulse wave signal amplitude of the user with a target pulse wave signal amplitude; and if the current pulse wave signal amplitude of the user is not less than the target pulse wave signal amplitude, start measurement and inflate the airbag to the preset pressure value at the preset rate.

In a possible implementation, the inflation module 52 may include a detection unit 621 and an inflation unit 622.

The detection unit 621 is configured to, if the user has changed from the first state to the second state, detect whether an acceleration in a direction opposite to gravity exists within a preset time.

The inflation unit 622 is configured to, if no acceleration in the direction opposite to gravity exists within the preset time, start measurement and inflate the airbag to the preset pressure value at the preset rate.

In a possible implementation, the obtaining module 53 may include a recording unit 531 and a calculating unit 532.

The recording unit 531 is configured to: record a first start moment of inflation to the airbag, and detect a pressure value of the airbag during inflation; and record a first end moment when the pressure value reaches the preset pressure value.

The calculating unit 532 is configured to determine the first duration based on a difference between the first end moment and the first start moment.

In a possible implementation, the apparatus 50 may further include a comparing module 56 and a prompt module 57.

The comparing module 56 is configured to compare the first duration with each of a first duration threshold and a second duration threshold.

The prompt module 57 is configured to: send a first signal if the first duration is less than or equal to the first duration threshold; and send a second signal if the first duration is greater than the second duration threshold.

In a possible implementation, the correction module 54 may include a calculating unit 541 and a correction unit 542.

The calculating unit 541 is configured to determine a first slope based on the preset pressure value and the first duration.

The correction unit 542 is configured to determine the correction value based on the first slope.

In a possible implementation, the apparatus 50 may further include a comparing module 66 and a prompt module 67.

The comparing module 66 is configured to compare the first slope with each of a first slope threshold and a second slope threshold.

The prompt module 67 is configured to: send a first signal if the first slope is greater than or equal to the first slope threshold; and send a second signal if the first slope is less than the second slope threshold.

The blood pressure measurement apparatus provided in the embodiment shown in FIG. 5 may be configured to perform the technical solutions of the method embodiment shown in FIG. 2 to FIG. 4 of this application. For implementation principles and technical effects thereof, refer to related descriptions in the method embodiment.

It should be understood that the division into modules of the blood pressure measurement apparatus shown in FIG. 5 is merely division of logical functions, and the modules may be all or partly integrated into one physical entity in actual implementation, or may be physically separated. These modules can all be implemented in a form of software invoked by a processing element. Alternatively, all of the modules are implemented in a form of hardware. Alternatively, some of the modules are implemented in a form of software invoked by a processing element, while some of the modules are implemented in a form of hardware. For example, the detection module may be an independently disposed processing element, or may be integrated into a chip of the electronic device for implementation. The implementation of other modules is similar to this. In addition, all or some of these modules may be integrated together, or may be implemented independently. In an implementation process, steps in the foregoing methods or the foregoing modules can be implemented by using a hardware integrated logical circuit in the processing element, or by using instructions in a form of software.

For example, these modules may be one or more integrated circuits configured to implement the foregoing method, for example, one or more application-specific integrated circuits (Application Specific Integrated Circuit, ASIC for short below), one or more microprocessors (Digital Signal Processor, DSP for short below), or one or more field programmable gate arrays (Field Programmable Gate Array, FPGA for short below). For another example, these modules may be integrated together for implementation in a form of a system-on-a-chip (System-On-a-Chip, SOC for short below).

In the foregoing embodiments, related processors may include, for example, a CPU, a DSP, a microcontroller, or a digital signal processor, and may further include a GPU, an embedded neural processing unit (Neural-network Process Units, NPU for short below), and an image signal processor (Image Signal Processing, ISP for short below). The processors may further include a necessary hardware accelerator or logic processing hardware circuit, for example, an ASIC, or one or more integrated circuits configured to control program execution of the technical solutions of this application. In addition, the processors may have a function of operating one or more software programs, and the software programs may be stored in a storage medium.

An embodiment of this application further provides a wearable device, including a processor 110, a pressure sensor 130, an air pump control circuit 140, an air pump 150, an airbag 160, and a signal receiver 170.

The signal receiver 170 is configured to detect a PPG signal and send the PPG signal to the processor 110.

The pressure sensor 130 is configured to detect a pressure value of the airbag 160 in a process of inflating the airbag 160 by the air pump 150, and send the pressure value to the processor 110.

The processor 110 is configured to: determine, based on the PPG signal, that the user has changed from a first state to a second state, indicate the air pump control circuit 140 to drive the air pump 150 to inflate the airbag 160 to the preset pressure value at a preset rate, and obtain first duration of the inflation process; determine a correction value based on the first duration; and after the measurement ends, obtain a measurement value of a blood pressure, and correct the measurement value based on the correction value, to obtain a final blood pressure value of the user.

In a possible implementation, the signal receiver 170 is further configured to obtain a current pulse wave signal amplitude of the user.

The processor 110 is further configured to: compare the current pulse wave signal amplitude of the user with a target pulse wave signal amplitude; and if the current pulse wave signal amplitude of the user is not less than the target pulse wave signal amplitude, notify the air pump control circuit 140 to drive the air pump 150 to inflate the airbag 160 to the preset pressure value at the preset rate.

In a possible implementation, the device further includes a motion sensor 180.

The motion sensor 180 is configured to detect whether an acceleration in a direction opposite to gravity exists within a preset time, and send a detection result to the processor 110.

The processor 110 is further configured to determine that no acceleration in the direction opposite to gravity exists within the preset time, and notify the air pump control circuit 140 to drive the air pump 150 to inflate the airbag 160 to the preset pressure value at the preset rate.

In a possible implementation, the processor is further configured to record a first start moment and a first end moment of inflating the airbag 160 by the air pump 150, and determine the first duration based on a difference between the first end moment and the first start moment.

In a possible implementation, the device further includes an audio circuit 190 and a vibrator 1100.

The audio circuit 190 is configured to give a voice prompt.

The vibrator 1100 is configured to vibrate for prompt.

The processor 110 is further configured to compare the first duration with each of a first duration threshold and a second duration threshold.

If the first duration is less than or equal to the first duration threshold, the processor indicates the audio circuit and/or the vibrator to send a first signal.

If the first duration is greater than the second duration threshold, the processor indicates the audio circuit and/or the vibrator to send a second signal.

In a possible implementation, the processor 110 is further configured to determine a first slope based on the preset pressure value and the first duration, and determine the correction value based on the first slope.

In a possible implementation, the device further includes an audio circuit 190 and a vibrator 1100.

The audio circuit 190 is configured to give a voice prompt.

The vibrator 1100 is configured to vibrate for prompt.

The processor is further configured to compare the first slope with each of a first slope threshold and a second slope threshold.

If the first slope is greater than or equal to the first slope threshold, the processor indicates the audio circuit and/or the vibrator to send a first signal.

If the first slope is less than the second slope threshold, the processor indicates the audio circuit and/or the vibrator to send a second signal.

In embodiments of this application, "at least one" means one or more, and "a plurality of means two or more. The term "and/or" describes an association relationship for describing associated objects and represents that three relationships may exist. For example, A and/or B may represent the following three cases: Only A exists, both A and B exist, and only B exists. A and B may be singular or plural. The character "/" usually indicates an "or" relationship between associated objects. "At least one of the following items" or a similar expression thereof indicates any combination of these items, including a single item or any combination of a plurality of items. For example, at least one of a, b, or c may indicate a, b, c, a and b, a and c, b and c, or a, b, and c, where a, b, and c may be singular or plural.

A person of ordinary skill in the art may be aware that, units and algorithm steps described in embodiments disclosed in this specification may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments, and details are not described herein again.

In several embodiments provided in this application, if any one of the functions is implemented in a form of a software function unit and sold or used as an independent product, the function may be stored in a computer-readable storage medium. Based on such an understanding, the technical solutions of this application essentially, or the part contributing to the conventional technology, or some of the technical solutions may be implemented in a form of a software product. The computer software product is stored in a storage medium, and includes several instructions for instructing a computer device (which may be a personal computer, a server, or a network device) to perform all or some of the steps of the methods described in embodiments of this application. The foregoing storage medium includes any medium that can store program code, such as a USB flash drive, a removable hard disk, a read-only memory (Read-Only Memory, ROM for short), a random access memory (Random Access Memory, RAM for short), a magnetic disk, or an optical disc.

The foregoing descriptions are merely specific implementations of this application. Any variation or replacement readily figured out by a person skilled in the art within the technical scope disclosed in this application shall fall within the protection scope in this application. Therefore, the protection scope of this application shall be subject to the protection scope of the claims.

## Claims

1. A blood pressure measurement method, comprising:
detecting a change of a user from a first state to a second state;
starting measurement and inflating an airbag to a preset pressure value at a preset rate if determining that the user has changed from the first state to the second state;
obtaining first duration of an inflation process;
determining a correction value based on the first duration; and
after the measurement ends, obtaining a measurement value of a blood pressure, and correcting the measurement value based on the correction value, to obtain a final blood pressure value of the user.

2. The method according to claim 1, wherein the starting measurement and inflating an airbag to a preset pressure value at a preset rate if determining that the user has changed from the first state to the second state further comprises:
obtaining a current pulse wave signal amplitude of the user if determining that the user has changed from the first state to the second state; and
comparing the current pulse wave signal amplitude of the user with a target pulse wave signal amplitude, and starting measurement and inflating the airbag to the preset pressure value at the preset rate if the current pulse wave signal amplitude of the user is not less than the target pulse wave signal amplitude.

3. The method according to claim 1 or 2, wherein the starting measurement and inflating an airbag to a preset pressure value at a preset rate if determining that the user has changed from the first state to the second state further comprises:
if determining that the user has changed from the first state to the second state, detecting whether an acceleration in a direction opposite to gravity exists within a preset time; and
if no acceleration in the direction opposite to gravity exists within the preset time, starting measurement and inflating the airbag to the preset pressure value at the preset rate.

4. The method according to any one of claims 1 to 3, wherein the obtaining first duration of an inflation process further comprises:
recording a first start moment of inflation to the airbag, and detecting a pressure value of the airbag during inflation;
recording a first end moment when the pressure value reaches the preset pressure value; and
determining the first duration based on a difference between the first end moment and the first start moment.

5. The method according to any one of claims 1 to 4, further comprising:
comparing the first duration with each of a first duration threshold and a second duration threshold;
sending a first signal if the first duration is less than or equal to the first duration threshold; and
sending a second signal if the first duration is greater than the second duration threshold.

6. The method according to any one of claims 1 to 3, wherein the determining a correction value based on the first duration further comprises:
determining a first slope based on the preset pressure value and the first duration; and
determining the correction value based on the first slope.

7. The method according to claim 6, further comprising:
comparing the first slope with each of a first slope threshold and a second slope threshold;
sending a first signal if the first slope is greater than or equal to the first slope threshold; and
sending a second signal if the first slope is less than the second slope threshold.

8. A wearable device, comprising a processor, a signal receiver, an air pump control circuit, an air pump, an airbag, and a pressure sensor, wherein
the signal receiver is configured to: detect a PPG signal and send the PPG signal to the processor;
the pressure sensor is configured to: detect a pressure value of the airbag in a process of inflating the airbag by the air pump, and send the pressure value to the processor; and
the processor is configured to: determine, based on the PPG signal, that a user has changed from a first state to a second state, indicate the air pump control circuit to drive the air pump to inflate the airbag to the preset pressure value at a preset rate, and obtain first duration of an inflation process; determine a correction value based on the first duration; and after the measurement ends, obtain a measurement value of a blood pressure, and correct the measurement value based on the correction value, to obtain a final blood pressure value of the user.

9. The device according to claim 8, wherein
the signal receiver is further configured to obtain a current pulse wave signal amplitude of the user; and
the processor is further configured to: compare the current pulse wave signal amplitude of the user with a target pulse wave signal amplitude, and if the current pulse wave signal amplitude of the user is not less than the target pulse wave signal amplitude, notify the air pump control circuit to drive the air pump to inflate the airbag to the preset pressure value at a preset rate.

10. The device according to claim 8 or 9, further comprising a motion sensor, wherein
the motion sensor is configured to: detect whether an acceleration in a direction opposite to gravity exists within a preset time, and send a detection result to the processor; and
the processor is further configured to: determine that no acceleration in the direction opposite to gravity exists within the preset time, and notify the air pump control circuit to drive the air pump to inflate the airbag to the preset pressure value at a preset rate.

11. The device according to any one of claims 8 to 10, wherein
the processor is further configured to: record a first start moment of inflation to the airbag and a first end moment; and determine the first duration based on a difference between the first end moment and the first start moment.

12. The device according to any one of claims 8 to 11, further comprising an audio circuit and a vibrator, wherein
the audio circuit is configured to give a voice broadcast;
the vibrator is configured to vibrate for prompt;
the processor is further configured to compare the first duration with each of a first duration threshold and a second duration threshold;
if the first duration is less than or equal to the first duration threshold, the processor indicates the audio circuit and/or the vibrator to send a first signal; and
if the first duration is greater than the second duration threshold, the processor indicates the audio circuit and/or the vibrator to send a second signal.

13. The device according to any one of claims 8 to 10, wherein
the processor is further configured to: determine a first slope based on the preset pressure value and the first duration; and determine the correction value based on the first slope.

14. The device according to claim 13, further comprising an audio circuit and a vibrator, wherein
the audio circuit is configured to give a voice broadcast;
the vibrator is configured to vibrate for prompt;
the processor is further configured to compare the first slope with each of a first slope threshold and a second slope threshold;
if the first slope is greater than or equal to the first slope threshold, the processor indicates the audio circuit and/or the vibrator to send a first signal; and
if the first slope is less than the second slope threshold, the processor indicates the audio circuit and/or the vibrator to send a second signal.
